# EUROPEAN PATENT SPECIFICATION

(11) **EP 1 395 599 B1**
(45) Date of publication and mention of the grant of the patent: **10.08.2005**
(21) Application number: 02730284.3
(22) Date of filing: 29.05.2002
(51) Int. Cl.: C07H 15/252, A61K 31/70, A61P 35/00

(54) **ANTI-TUMOUR ANTHRACYCLINE GLYCOSIDE 14-SULFONIC ACID DERIVATIVES**
ANTHRACYCLINGLYKOSID-14-SULFON SÄUREDERIVATE MIT ANTITUMORWIRKUNG
DERIVES ANTITUMORAUX DE L'ACIDE SULPHONIQUE-14 DE GLYCOSIDES D'ANTHRACYCLINES

(30) Priority: 15.06.2001 GB 0114654
(43) Date of publication of application: 10.03.2004
(73) Proprietor: Pharmacia Italia S.p.A., 20152 Milano (IT)
(72) Inventor: ARCARI, Alessandro, I-20148 Milano (IT); GIGLI, Mauro, I-20141 Milano (IT)
(74) Representative: Modiano, Micaela Nadia
(86) International application number: PCT/EP2002/005907
(87) International publication number: WO 2002/102817

(56) References cited:
- WO-A-01/05382
- WO-A-01/05425
- WO-A-96/04895
- GB-A- 2 169 286
- GB-A- 2 195 998
- US-A- 4 098 884

## Description

An antitumor composition comprising an alkylating anthracycline and a recombinant humanized anti-HER 2 antibody having a synergistic or additive anti-neoplastic effect is known from WO-A2-01/05425. WO A1-01/05382 discloses an antitumor composition comprising an alkylating anthracycline and an antimetabolite compound having a synergistic or additive antineoplastic effect.

The invention relates to anthracycline glycosides, to a process for their preparation and to pharmaceutical compositions containing them.

The invention provides a compound which is an anthracycline glycoside of the formula I: 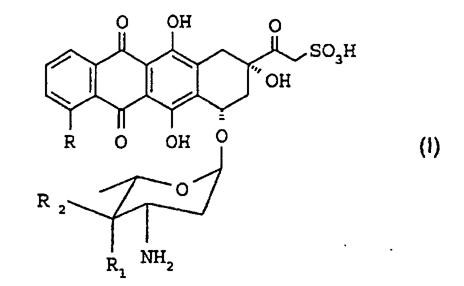 wherein R is hydrogen, hydroxy or methoxy, R₁ and R₂, which are the same or different, are independently hydrogen or hydroxy, or a pharmaceutically acceptable salt thereof.

In preferred compounds of formula I R is methoxy, R₁ is hydroxy and R₂ is hydrogen; or R is methoxy, R₁ is hydrogen and R₂ is hydroxy. These compounds are
- daunorubicin 14-sulfonic acid (Ia) and
- 4' epi daunorubicin 14-sulfonic acid (Ib).

The compounds of formula (I) may exist as zwitterions, i.e. as internal salts of a 14-SO₃-group with the amino group on the sugar residue; such salts are also part of the present invention.

The pharmaceutically acceptable salts are, for example, alkali metal and alkaline earth metal salts (e.g. sodium, potassium, lithium, calcium and magnesium salts), ammonium salts and salts with an appropriate organic amine or amino acid (e.g. arginine, procaine salts), and the addition salts formed with suitable organic or inorganic acids (e.g. hydrochlorides, hydrobromides, sulfates, phosphates) or carboxylic and sulfonic organic acids (e.g. acetates, citrates, succinates, malonates, lactates, tartrates, fumarates, maleates, methanesulphonates, *p*-toluenesulphonates).

The compounds of the formula I may be prepared by reacting the anthracycline of the formula II 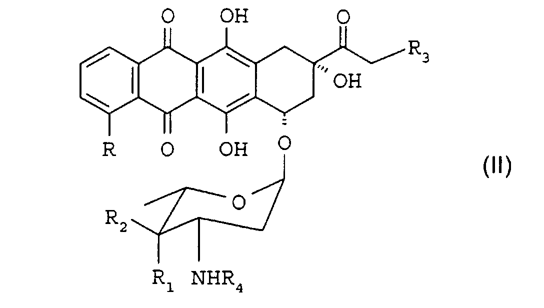 wherein R, R₁ and R₂ are as defined above, R₃ is hydroxy or a leaving group, and R₄ is hydrogen or a nitrogen protecting group, with a sulfiting agent in a polar solvent or a mixture of polar solvents, and, if desired, removing the protecting groups from the resulting compound. The resulting compound may be converted into a pharmaceutically acceptable salt thereof. Suitable leaving group which R₃ may represent include halogen atoms, such as bromine, iodine or chlorine, or mesyl, trifluoromesyl or p-toluensulfonyl groups. Suitable nitrogen protecting groups include, for instance, a trifluoroacetyl group. Examples of suitable solvents include acetone, water and mixtures of acetone and water. The reaction is preferably conducted at a temperature below 50°C, more preferably at room temperature. The desired resultant derivative may be isolated by precipitation from appropriate solvent mixtures. The starting material for the preparation of the new anthracycline glycosides is an optionally protected daunorubicin or 4-demethoxy daunorubicin, that may be easily converted into the compounds of the formula II as described in Antitumor Anthracyclines, Chim. Oggi, April 1990, p. 9-19.

The invention further provides a pharmaceutical composition comprising an anthracycline glycoside of formula I in admixture with a pharmaceutically acceptable diluent or carrier. Conventional carriers and diluents may be used. The composition may be formulated and administered in conventional manner.

The suspension or solutions for intramuscular injections may contain, together with the active compound, a pharmaceutically acceptable carrier, e.g. sterile water, olive oil, ethyl oleate, glycols, e.g. propylene glycol, and, if desired, a suitable amount of lidocaine hydrochloride. The solutions for intravenous injections or infusions may contain as carrier, for example, sterile water or preferably they may be in the form of sterile, aqueous, isotonic saline solutions or they may contain as a carrier propylene glycol.

The compounds according to the invention are useful in methods of treatment of the human or animal body by therapy. They are useful as anti-tumor agents. They are useful in the treatment of leukemia and solid tumors, such as colon, colon-rectal, ovarian, mammary, prostate, lung, kidney and also melanoma tumors. A human can therefore be treated by a method comprising administering thereto a therapeutically effective amount of a compound of the invention. The condition of the human patient can thus be improved. The dosage to be given can be ascertained using known dosage ranges in the field of anthracyclines, modified by reference to the activity shown by the present compounds in *in vitro* and *in vivo* anti-tumor tests. Suitable dosages are generally in the range of 1 to 200 mg/m² body surface, preferably from 1 to 100 mg/m², depending on the nature and severity of the disease being treated and on the general condition of the patient.

The compounds of formula I were tested and found active *in vitro* against a tumor cell lines, and *in vivo* on murine leukemia.

On turnor cell lines, the compounds of the present invention were shown to present high cytotoxicity. The *in vivo* tests were carried on murine leukemia.

### Example 1

### Daunorubicin 14-sulfonic acid (Ia)

14-bromo-daunorubicin hydrochoride (II, R=OCH₃, R₁=OH, R₂ = H, R₃ = Br, 2mmol) were dissolved in acetone-water (30+30ml). The solution was added with an aqueous solution of sodium sulfite (570 mg, 4.38 mmol), and the pH was adjusted to about 7.6 with aqueous ammonia 10%. After stirring 3 hours at room temperature, the reaction mixture was concentrated to dryness. The residue was suspended in methanol, and the methanolic suspension was dropped in acetone at 4-5EC. The precipitate was filtered, washed on the filter and dried under vacuum. The product has been identified by MS and NMR analysis.

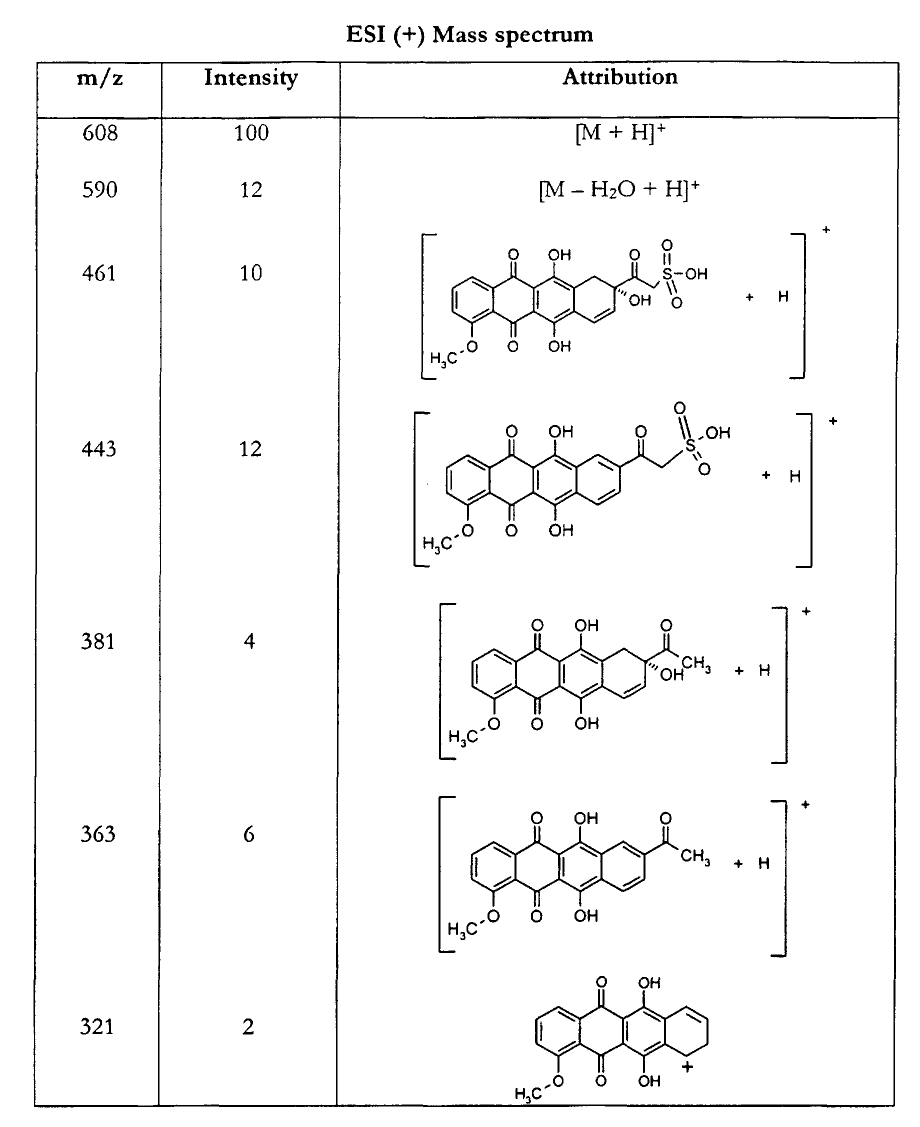

### High resolution mass spectrometry.

An exact mass determination has been performed on the protonated molecular ion at m/z 608, with the following result:
Calculated for C₂₇H₂₉NO₁₃S + H: 608.14379 Found: 608.14374 Δ = 0.0 ppm

| ^{**1**}**H NMR chemical shifts and coupling constants** | | | |
|---|---|---|---|
| **δ (ppm)** | **Multiplicity*** | **J (Hz)** | **Proton** |
| 1.11 | d | 6.5 | CH₃-6' |
| 1.62 | dd | 12.5,4.2 | H-2'eq |
| 1.83 | ddd | 12.5, 12.5, 3.4 | H-2'ax |
| 2.03 | dd | 14.8,5.3 | H-8β |
| 2.36 | d | 14.8 | H-8α |
| 2.73 | d | 18.0 | H-10β |
| 2.99 | d | 18.0 | H-10α |
| 3.25^{◆} | dd^{◆} | 12.5, 4.2^{◆} | H-3' |
| 3.50 | s | - | H-4' |
| 3.86 | d | 10.9 | H-14a |
| 3.99 | s | - | OCH₃ |
| 4.18 | d | 10.9 | H-14b |
| 4.33 | q | 6.5 | H-5' |
| 4.90 | d | 5.3 | H-7 |
| 5.30 | d | 3.4 | H-1' |
| 6.13 | s | - | OH-9 |
| 7.66 | m | - | H-3 |
| 7.92 | m | - | H-2, H-1 |

| | | | |
|---|---|---|---|
| * s= singlet, d-doublet, t=triplet, q=quartet, m-multiplet | | | |
| ◆ H₂O signal overlaps this resonance; chemical shift was detected in 2D experiments and coupling constants were measured on J-coupled signals. | | | |

| ^{**13**}**C NMR chemical shifts*** | | | |
|---|---|---|---|
| δ (ppm) | Carbon | δ (ppm) | Carbon |
| 17.1 | C-6' | 99.5 | C-1' |
| 29.3 | C-2' | - | C-5a, C-11a^{▼} |
| 33.2 | C-10 | 119.5 | C-1 |
| 36.7 | C-8 | 120.2 | C-3, C-4a |
| 47.2 | C-3' | 135.0, 136.0 | C-10a, C-6a |
| 57.0 | OCH₃ | 135.1 | C-12a |
| 61.5 | C-14 | 136.7 | C-2 |
| 66.6 | C-5' | - | C-11, C-6^{▼} |
| 67.2 | C-4' | 161.1 | C-4 |
| 69.5 | C-7 | - | C-5, C-12^{▼} |
| - | C-9^{▼} | 208.6 | C-13 |

| | | | |
|---|---|---|---|
| * Carbon chemical shift were detected in the HSQC and GHMBC experiments | | | |
| ^{▼} no correlations were observed in the GHMBC experiment for these carbons | | | |

## Claims

1. A compound which is an anthracycline glycoside of the formula I: 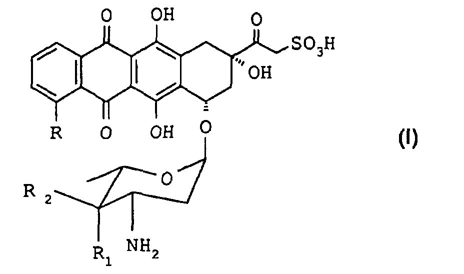 wherein R is hydrogen, hydroxy or methoxy, R₁ and R₂, which are the same or different, are independently hydrogen or hydroxy; or a pharmaceutically acceptable salt thereof.

2. A compound according to claim 1 which is daunorubicin 14-sulfonic acid or 4'epi daunorubicin 14-sulfonic acid.

3. A compound according to claim 1 which is daunorubicin 14-sulfonic acid.

4. A process for producing an anthracycline glycoside of formula (I) as defined in claim 1, which process comprises reacting an anthracycline of the formula II 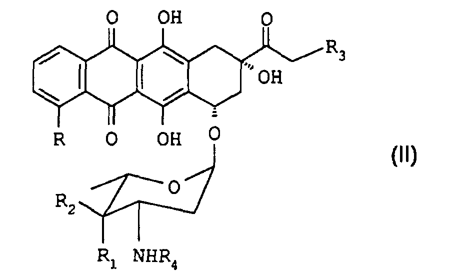 wherein R, R ₁ and R₂ are as defined in claim 1, R₃ is hydroxy or a leaving group, and R₄ is hydrogen or a nitrogen protecting group, with a sulfiting agent, in a polar solvent or a mixture of polar solvents and, if desired, removing the protecting groups from the resulting compound.

5. A process according to claim 4 which further comprises converting the resulting compound into a pharmaceutically acceptable salt thereof.

6. A pharmaceutical composition comprising a compound as defined in claim 1, and a pharmaceutically acceptable carrier or diluent.

7. A compound according to any one of claims 1 to 3, for use in a method of treatment of the human or animal body by therapy.

8. A compound as claimed in claim 7 for use as an antitumor agent.

9. Use of a compound as defined in any one of claims 1 to 3 in the manufacture of a medicament for the treatment of tumors.

10. Use of a compound as defined in any one of claims 1 to 3, for the manufacture of a medicament for treating a patient in need of an antitumor agent.

## Patentansprüche

1. Eine Verbindung, welche ein Anthracyclinglycosid mit der Formel I ist: 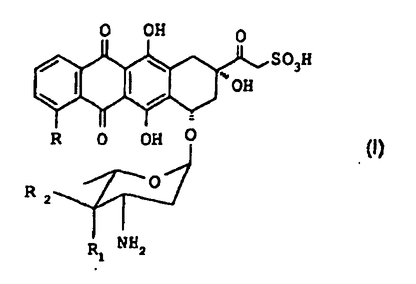 worin R Wasserstoff, Hydroxy oder Methoxy ist, R₁ und R₂, welche gleich oder unterschiedlich sind, sind unabhängig Wasserstoff oder Hydroxy; oder ein pharmazeutisch verträgliches Salz davon.

2. Eine Verbindung gemäß Anspruch 1, welche Daunorubicin 14-Sulfonsäure oder 4'epi Daunorubicin 14-Sulfonsäure ist.

3. Eine Verbindung gemäß Anspruch 1, welche Daunorubicin 14-Sulfonsäure ist.

4. Ein Verfahren zur Herstellung eines Anthracyclinglycosids der Formel (I), wie in Anspruch 1 definiert, wobei das Verfahren die Reaktion eines Anthracyclins der Formel II umfaßt 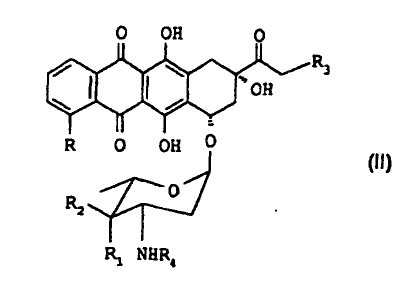 worin R, R₁ und R₂ wie in Anspruch 1 definiert sind, R₃ ist Hydroxy oder eine Abgangsgruppe, und R₄ ist Wasserstoff oder eine Stickstoffschutzgruppe, mit einem Sulfitierungsmittel, in einem polaren Lösungsmittel oder einer Mischung aus polaren Lösungsmitteln und, wenn gewünscht, Entfernen der Schutzgruppen von der resultierenden Verbindung.

5. Ein Verfahren gemäß Anspruch 4, welches weiter das Umwandeln der resultierenden Verbindung in ein pharmazeutisch verträgliches Salz davon umfaßt.

6. Eine pharmazeutische Zusammensetzung, die eine Verbindung, wie in Anspruch 1 definiert, und einen pharmazeutisch verträglichen Träger oder Verdünnungsmittel umfaßt.

7. Eine Verbindung gemäß irgendeinem der Ansprüche 1 bis 3, für die Verwendung in einem Verfahren zur Behandlung des menschlichen oder tierischen Körpers durch Therapie.

8. Eine Verbindung wie in Anspruch 7 beansprucht, für die Verwendung als ein Antitumormittel.

9. Verwendung einer Verbindung wie in irgendeinem der Ansprüche 1 bis 3 definiert, zur Herstellung eines Medikaments für die Behandlung von Tumoren.

10. Verwendung einer Verbindung wie in irgendeinem der Ansprüche 1 bis 3 definiert, zur Herstellung eines Medikaments für die Behandlung eines Patienten, der ein Antitumormittel benötigt.

## Revendications

1. Composé qui est un glycoside de la classe des anthracyclines de formule I : 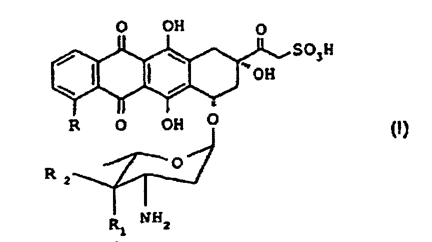 où R est l'hydrogène, un groupe hydroxyle ou méthoxy, R₁ et R₂, qui sont identiques ou différents, sont indépendamment l'hydrogène ou un groupe hydroxyle ; ou un sel pharmaceutiquement acceptable de celui-ci.

2. Composé selon la revendication 1, qui est l'acide daunorubicine-14-sulfonique ou l'acide 4'-épidaunorubicine-14-sulfonique.

3. Composé selon la revendication 1, qui est l'acide daunorubicine-14-sulfonique.

4. Procédé de production d'un glycoside de la classe des anthracyclines de formule (I) selon la revendication 1, lequel procédé comprend la réaction d'une anthracycline de formule II 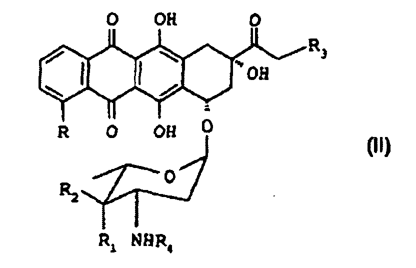 où R, R₁ et R₂ sont tels que définis dans la revendication 1, R₃ est un groupe hydroxyle ou un groupe partant, et R₄ est l'hydrogène ou un groupe protecteur d'azote, avec un agent sulfitant, dans un solvant polaire ou un mélange de solvants polaires et, éventuellement, l'élimination des groupes protecteurs du composé résultant.

5. Procédé selon la revendication 4, qui comprend en outre la conversion du composé résultant en un sel pharmaceutiquement acceptable de celui-ci.

6. Composition pharmaceutique comprenant un composé tel que défini dans la revendication 1, et un support ou diluant pharmaceutiquement acceptable.

7. Composé selon l'une quelconque des revendications 1 à 3, pour son utilisation dans un procédé de traitement du corps humain ou animal par thérapie.

8. Composé selon la revendication 7, pour son utilisation comme agent antitumoral.

9. Utilisation d'un composé selon l'une quelconque des revendications 1 à 3 dans la fabrication d'un médicament destiné au traitement de tumeurs.

10. Utilisation d'un composé selon l'une quelconque des revendications 1 à 3, pour la fabrication d'un médicament destiné à traiter un patient ayant besoin d'un agent antitumoral.
